# EUROPÄISCHE PATENTSCHRIFT

(11) **EP 0 890 364 B1**
(45) Veröffentlichungstag und Bekanntmachung des Hinweises auf die Patenterteilung: **01.10.2003**
(21) Anmeldenummer: 97810461.0
(22) Anmeldetag: 11.07.1997
(51) Int. Cl.: A61L 9/12, F16H 31/00

(54) **Vorrichtung und Verfahren zur Abgabe von flüssigen Wirkstoffen durch Verdunstung**
Device and process for dispensing liquid agents by evaporation
Dispositif et procédé pour diffuser des agents liquides par évaporation

(43) Veröffentlichungstag der Anmeldung: 13.01.1999
(73) Patentinhaber: HTS International Trading AG, CH-6340 Baar (CH)
(72) Erfinder: Studer, Hans-Jörg, 8335 Hittnau (CH); Ehrensperger, Markus, Dr., 8442 Hettlingen (CH)
(74) Vertreter: Frauenknecht, Alois J.

(56) Entgegenhaltungen:
- WO-A-80/00792
- WO-A-94/04280
- GB-A- 2 222 775
- US-A- 4 078 891
- US-A- 4 780 253

## Beschreibung

Die vorliegende Erfindung bezieht sich auf eine Vorrichtung gemäss dem Oberbegriff des Patentanspruchs 1 sowie auf Verfahren zu deren Betrieb.

Grundsätzlich lassen sich desodorierende Spender (auch Luftverbesserer genannt) in vier Kategorien einteilen: In solche mit natürlicher Konvektion des Wirkstoffes (engl. air freshening material), in solche mit mechanischer Unterstützung der Zirkulation, in solche mit thermisch verstärkter Verdunstung der flüchtigen Komponenten des Stoffs und in Raumsprayspender, welche über eine schlagartig betätigte Pumpe eine feine Tröpfchenwolke verbreiten (EP -A1- 0 127 573 und WO 94/04280).

Diese letztgenannten Spender versprühen kurzzeitig eine intensive Duftwolke und neigen in kleinen Räumen, aufgrund ihrer Montage an oder bei einer Türe, dazu die einen Raum betretenden oder verlassenden Personen zu parfümieren.

Die US -A- 4,078,891 beschreibt einen Luftreiniger, welcher am Stromnetz angeschlossen ist. Die zu reinigende Luft wird durch einen Ventilator über ein Filter angesogen und im Inneren des Gehäuses mit Wirkstoffen angereichert und wieder ausgeblasen. Diese Anreicherung erfolgt über in Flaschen eingetauchte Dochte und/oder Verdunstungstabletten, welche auf einem Gitter gelagert sind. Unterhalb des Gitters befindet sich der relativ grosse, netzbetriebene Ventilator sowie eine zugehörige Steuerelektronik, welche diesen vorgegeben, sequentiell ein- und ausschaltet.

Nachteilig sind bei dieser Anordnung, dass keine bedarfsabhängige Steuerung erfolgt und ein Netzanschluss notwendig ist, der in Nassräumen, wie Toiletten etc. ein Sicherheitsrisiko darstellt und besondere teure Installationen erfordert. Das Innere des Gehäuses wird zudem, auch bei ausgeschaltetem Ventilator über das Filter und die Ausströmleitung von Aussenluft durchströmt, so dass sich der dort enthaltene Wirkstoff laufend abbaut. - Entsprechend gross ist auch der Wirkstoffverbrauch; die notwendigen Service-Intervalle sind kurz.

Es ist daher Aufgabe der vorliegenden Erfindung eine bedarfsabhängig gesteuerte, hygienische Vorrichtung zu schaffen, welche jedes Besprühen von Personen, auch in kleinen Räumen, vermeidet und zudem wirtschaftlich im Betrieb und einfach im Unterhalt ist. Die Vorrichtung soll einen geringen Installationsaufwand aufweisen und insbesondere auch bestehende Infrastrukturen, ohne oder nur mit geringem Anpassungsaufwand nutzen können.

Im weiteren soll die Vorrichtung möglichst universell einsetzbar und insbesondere für Toiletten-, Pissoir- und Waschräume geeignet sein. Die Lärmentwicklung der Vorrichtung soll minimal sein, so dass diese problemlos auch an anderen Erten Verwendung finden kann, wie beispielsweise in Warteräumen, Aufzügen, Telefonkabinen etc.

Diese Aufgabe wird nach den Merkmalen des Anspruchs 1 gelöst.

Die Vorrichtung nach Anspruch 1 geht von der Erkenntnis aus, dass der Bedarf an grösseren Mengen an desodorierenden Wirkstoffen in zeitlichen Abständen erfolgt, so dass sich im Innern der Vorrichtung, jeweils zwischen den Intervallen der Verströmung, eine gesättigte Atmosphäre aufbauen kann.

Ebenfalls nutzt die erfindungsgemässe Vorrichtung die natürliche - temperaturabhängige - Verdunstungsleistung der Wirkstoffe in einem quasi geschlossenen Verdunstungs-Raum, welcher je nach Bedürfnis, bzw. Benutzung der Umgebung und ihrer Infrastruktur (begehbarer Raum; Toilette etc.) "gelüftet" wird, d.h. dessen mit Wirkstoff gesättigte Atmosphäre durch Aussen- oder Umgebungsluft ersetzt wird.

In weiteren abhängigen Ansprüchen sind vorteilhafte Weiterbildungen des Erfindungsgegenstands beschrieben.

Die Zuordnung von drei definierten Positionen und Funktionen zur Stellung des Schwenkhebels, gemäss Anspruch 2, ist kinematisch günstig und erfordert nur geringe Schwenkbewegungen und kleine Kräfte.

Der Einbezug eines an sich bekannten Federmotors nach Anspruch 3, erlaubt eine Impulsleistung zu speichern und zu beliebiger Zeit dosiert an den Verdichter abzugeben.

Durch ein Umschaltjoch gemäss Anspruch 4, kann eine beliebige Schwenkbewegung, unabhängig von ihrem Drehsinn ausgenutzt werden; d.h. die Drehrichtung des Verdichters bleibt unabhängig vom Drehsinn des Schwenkhebels.

Bewährt hat sich ein Zug-/Druckventilator nach Anspruch 5, welcher energetisch günstig ist und ein gerichtetes Durchspülen des Verdunstungs-Raums bewirkt. Zusätzlich wirken die beiden strömungstechnisch voneinander getrennten Zug- und Druckkammern bei stillstehendem Laufrad als Labyrinth-Dichtungen und verhindern einen unkontrollierten Austausch der mit Wirkstoff gesättigten Atmosphäre an die Umgebung.

Zwei gemäss Anspruch 6 angeordnete Vorratsflaschen mit entsprechenden Verdunstungselementen, ergeben einen unterbruchsfreien Betrieb, da während den üblichen Serviceintervallen sich zuerst eine Flasche leert und die zweite noch einen Vorrat an Wirkstoff enthält, bevor man sie an die Stelle der ersten Flasche plaziert und an ihren Platz eine neue, volle Ersatz-Flasche einsetzt.

Die Verdunstungselemente sind hygienisch einwandfrei und lassen sich problemlos entsorgen; die Vorratsflaschen sind wiederverwendbar.

Durch den Einbezug von Blenden bzw. Schiebern, Ansprüche 7 bzw. 9, lässt sich das zulässige oder gewünschte austretende Volumen an verdunstetem Wirkstoff, beispielsweise abhängig von der Umgebungstemperatur und Raumgrösse einstellen. Dies ergibt eine Anpassung der "Grundbeduftung", welche grundsätzlich bereits durch die Leckage (Undichtigkeit) am stillstehenden Laufrad des Ventilators entsteht.

Ein oberhalb des Gerätegehäuses angeordneter Lufteinlass ist strömungstechnisch und in Bezug auf Staubeinwirkungen günstig, vgl. Anspruch 8.

Besonders einfach und wirtschaftlich herzustellen ist ein Laufrad gemäss Anspruch 10.

Die Verfahren nach den Ansprüchen 11 und 12 nutzen die Bewegungsenergie von Türbewegungen und übertragen diese teilweise auf den Schwenkhebel. Das Verfahren nach Anspruch 11 ist für an der Türe selbst montierte Vorrichtungen und das Verfahren nach Anspruch 12 für solche an einem Türrahmen angeordnete, bestimmt.

Im folgenden werden anhand von Zeichnungen Ausführungsbeispiele des Erfindungsgegenstands erläutert.

Es zeigen:
- Fig. 1: eine Vorrichtung mit abgenommenem Gehäusedeckel und einem Verdichter, zusammen mit dessen Antrieb mit Schwenkhebel,
- Fig. 2: eine Darstellung der Vorrichtung nach Fig. 1, mit herausgenommenen Vorratsflaschen, und weiteren Einzelheiten, in einer Schrägansicht von unten,
- Fig. 3: das Laufrad des Ventilators Fig. 1 und 2,
- Fig. 4: einen Gehäusedeckel mit Lufteinlass und Ausblasund Austrittsöffnungen,
- Fig. 5: eine Draufsicht auf den Antrieb Fig. 1 und 2, in Perspektivdarstellung, bei geöffnetem Getriebegehäuse,
- Fig. 6: das vereinfacht dargestellte Getriebe Fig. 5 für eine Betätigung des Schwenkhebels durch eine rechtsbündige Türe und
- Fig. 7: das Getriebe Fig. 6 mit umgestelltem Schwenkhebel für eine Betätigung durch eine linksbündige Türe.

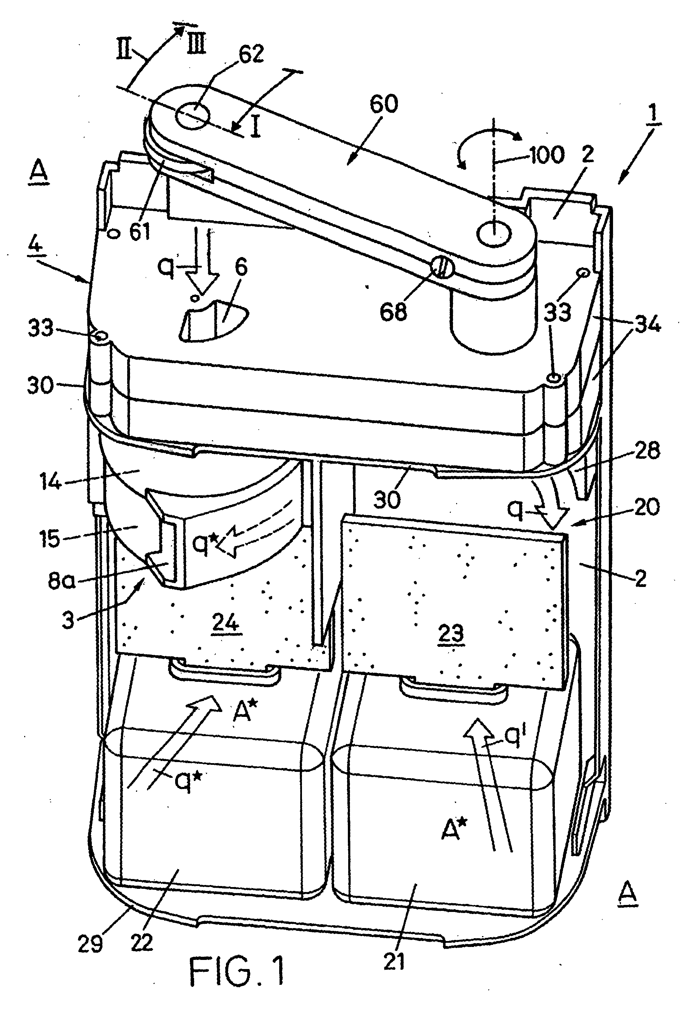

In Fig. 1 ist mit 1 ein Dispenser zum bedarfgerechten verdunsten von Wirkstoff bezeichnet. Die Darstellung Fig. 1 ist mit abgenommenen Gehäusedeckel gezeichnet und daher in dieser Form nicht gebrauchsfähig.

Der Dispenser 1 basiert auf einer Montageplatte 2, welche bevorzugter Weise an einer Wand oder einer Türe fixiert ist. Im oberen linken Quadranten befindet sich ein Verdichter 3, ein Zug-/Druckventilator mit zwei Kammern. Oberseitig am Verdichter 3 ist dessen Antrieb 4, ein Federmotor zu sehen. Aus einem zweiteiligen Getriebegehäuse 34 des Federmotors 4 ragt eine Drehachse 100, auf welcher ein Schwenkhebel 60, kraftschlüssig verbunden, aufgesetzt ist.

Der Schwenkhebel 60 besitzt an seinem vorderen Ende, mittig angeordnet eine Auslöserrolle 61 mit Lagerzapfen 62, wobei die Rolle 61 die äussere Kontur überragt. Stirnseitig ist im Bereich der Drehachse 100 eine Verstellschraube 68 zu sehen, die in bekannter Weise zur Grundeinstellung des Winkels zwischen dem Schwenkhebel 60 und der rückseitigen Auflagefläche der Montageplatte 2 dient.

In Fig. 1 sind ebenfalls die funktionellen Positionen und Bewegungen des Schwenkhebels 60 zu sehen, wobei die:

III. Position die Ruhelage bedeutet; eine Bewegung im Gegenuhrzeigersinn, in Pfeilrichtung, führt zur Position I., dabei wird der Antrieb 4 gespannt; die auf den Verdichter wirkende Entspannung ist mit II. bezeichnet.

Im weiteren ist ein Durchbruch im Getriebegehäuse 34 eingezeichnet, der als Zuluftkanal 6 für den Luftstrom q benutzt ist.

Im unteren Teil des Verdichters 3 ist ein Volumenstrom q* eingezeichnet, welcher aus dessen unterer Kammer 15 eine gesättigte Atmosphäre A* in Richtung Ausblasöffnung 8a fördert.

Unterhalb des Antriebs 4 ist rechts ein weiterer Zuluftkanal 28 angedeutet, der die obere Kammer 14 des Verdichters 3 mit dem Verdunstungs-Raum 20 verbindet.

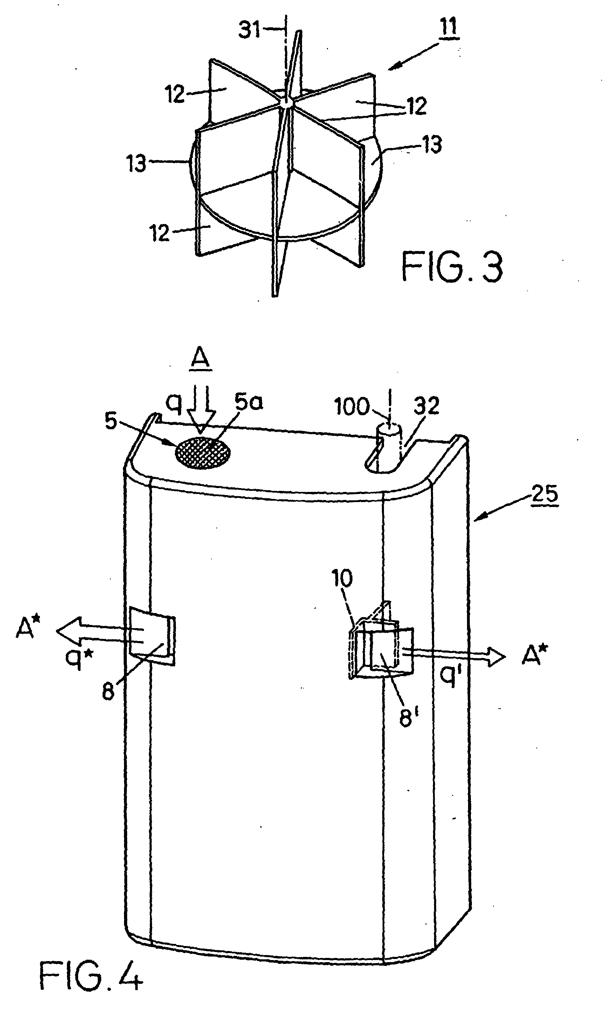

Sobald ein Gehäusedeckel 25, vgl. Fig. 4, auf die Montageplatte 2 aufgestülpt ist, bildet sich im innern des Dispensers 1 der Verdunstungs-Raum 20, der über flächige Verdunstungselemente 23, 24 und Vorratsflaschen 21, 22 laufend mit einem handelsüblichen Wirkstoff beschickt wird und somit die vorgängig erwähnte gesättigte Atmosphäre A* einschliesst.

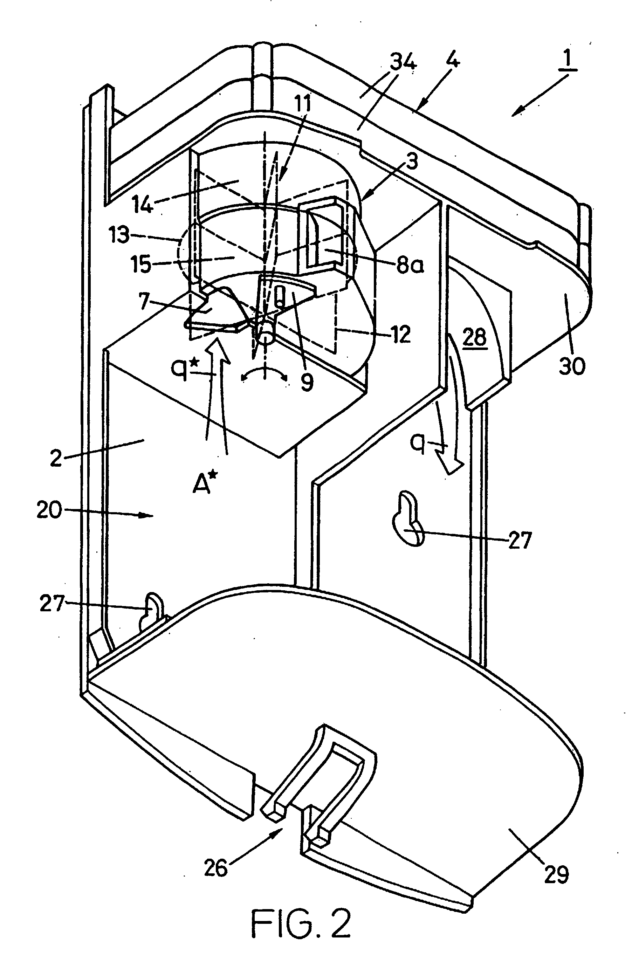

Der Dispenser 1 ist mit weiteren Einzelheiten in Fig. 2 dargestellt.

Hier ist u.a. ersichtlich, wie der Verdichter 3 aufgebaut ist und wie ein darin befindliches einziges Laufrad 11 mit seinen Schaufeln 12 in Kammern 14 und 15 angeordnet ist.

Die notwendige Trennung zwischen dem Zug- und dem Druckteil erfolgt am Laufrad 11 durch eine radiale Trennwand 13 welche ebenfalls angedeutet ist.

Unterhalb der Kammer 15 ist eine Absaugöffnung 7 für die angereicherte Atmosphäre A* vorgesehen, wobei die Absaugöffnung mittels einer Blende 9, an einem Hebel in Pfeilrichtung drehbar, in einfachster Weise eine Einstellung des Volumenstroms q* zulässt. Der Weg des Volumenstroms q mit der zugeführten Luft in den Raum 20 ist ebenfalls ersichtlich; er wird durch das Druckteil des Verdichters 3, über dessen oben partiell offene Kammer 14 geführt.

Ebenfalls sind zwei zu einander diagonal angeordnete Langlöcher 27, welche der Befestigung des Dispensers dienen in der Montageplatte 2, zu sehen. Unterhalb des die Vorratsflaschen 21, 22 tragenden Supports 29 befindet sich eine Deckel-Verriegelung 26, die bei aufgesetztem Gehäusedeckel 25 in diesem einrastet.

Im einzelnen ist das Laufrad 11 in Fig. 3 aufgezeigt. Wiederum ist die radiale Trennwand 13 zu sehen, welche die Schaufeln 12 in zwei Kammern teilt. Die Drehachse 31 ist lediglich angedeutet, sie ist die Motorachse des Antriebs 4, welche direkt in das Laufrad 11 eingesetzt ist.

Der Gehäusedeckel 25 in Fig. 4 weist einen Durchführungsschlitz 32 auf, durch welchen eine Drehachse 100, auf welcher der Schwenkhebel 60 aufgesetzt ist, durchragt. Auf der oberen Stirnseite dieses Gehäusedeckels 25 befindet sich zusätzlich, links ein Lufteinlass 5 mit einem eingelegten Filtersieb 5a.

Frontseitig am Gehäusedeckel 25 sind eine Ausblasöffnung 8 und eine Austrittsöffnung 8' vorgesehen. Hinter der schlitzartigen Austrittsöffnung 8' befindet sich ein Schieber 10 der zur Einstellung des Volumenstroms q', zur Anpassung der Grundbeduftung, herangezogen wird.

Die Volumenströme q aus der Umgebungsluft A bzw. die gesättigten Volumenströme q' und q* sind mit der entsprechenden Atmosphäre A* beladen, wie eingezeichnet, und wirken bei einer Aktivierung des Ventilators 3 gleichzeitig.

Aus Fig. 1, in Verbindung mit Fig. 2 erkennt man, dass die horizontale Trennfläche 30 den Dispenser in eine obere, und eine untere als Verdunstungs-Raum 20 dienende Kammer aufteilt.

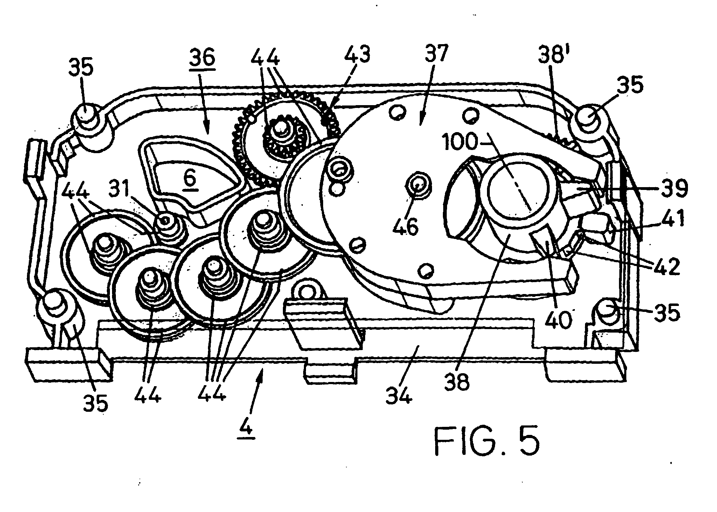

Die Darstellung Fig. 5 zeigt in vereinfachter Form den Federmotor 4 mit Getriebe 36 in seinem geöffneten Getriebegehäuse 34 mit teilweise vereinfacht dargestellten Zahnrädern 44.

Der hier nicht dargestellte Schwenkhebel betätigt über die Drehachse 100 eine Nockenhülse 38 mit ihren beiden Stellnocken 39 und 40. Einer der beiden Nocken 39 oder 40 wird je nach festgelegter Drehrichtung ein Umschaltjoch 37 um seine Drehachse 46 schwenken und über einen hier nicht ersichtlichen Zahnkranz und weitere Zahnräder den eigentlichen Federmotor 43, eine Spiralfeder in einem Gehäuse aufziehen, d.h. zu spannen.

Die weiteren Zahnräder 44 dienen der Übersetzung der Bewegung beim Entspannen der Spiralfeder und aktivieren schliesslich über die Drehachse 31 den vorgängig diskutierten Ventilator 3.

Der mögliche Schwenkbereich der Nockenhülse 38 mit ihrem Zahnsegment 38' wird durch einen feststehenden Anschlag 41 und den beiden Begrenzungs-Nocken 42 begrenzt.

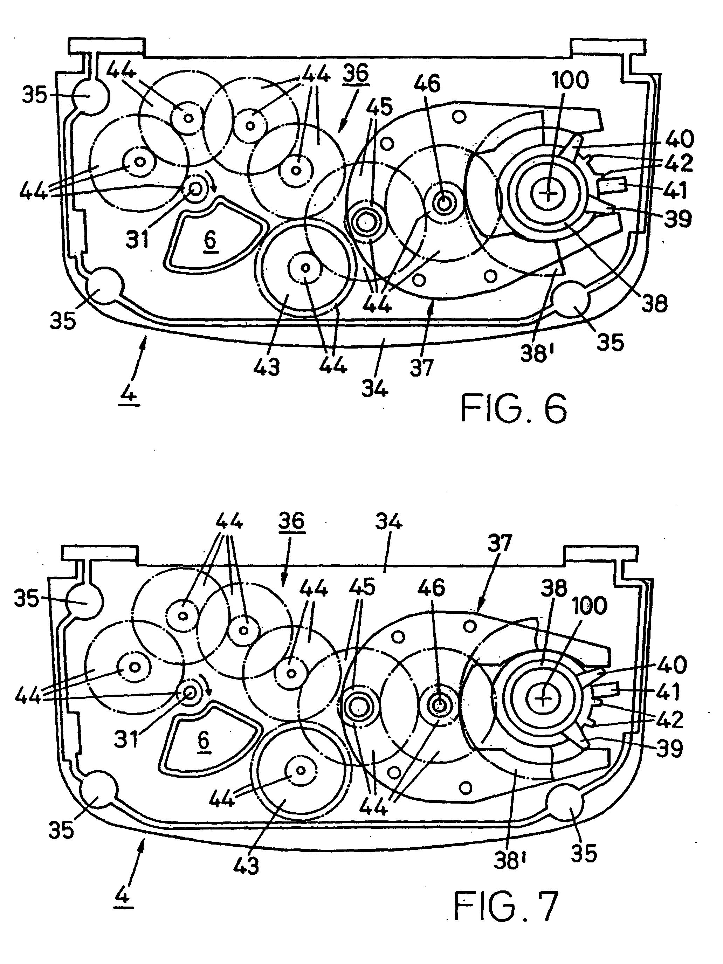

Die Funktionsweise des Getriebes 36 ist mit weiteren Einzelheiten aus den Fig. 6 und 7 zu entnehmen.

Das hier bezeichnete Zwischengetriebe 45 bewirkt eine Drehrichtungsumkehr, so dass ungeachtet, ob die Betätigung des Schwenkhebels 60 durch eine rechtsbündige Türe, entsprechend der Stellung des Zwischengetriebes nach Fig. 6 oder durch eine linksbündige Türe mit dem Antrieb entsprechend Fig. 7 erfolgt.

Das Getriebegehäuse 34 ist zweiteilig und weist an seinen Eckpunkten Zentrierzapfen 35 auf, welche in die gegenüberliegende Gehäusehälfte eingreifen und in bekannter Weise mittels Hohlnieten 33, Fig. 1, miteinander fixiert sind.

Der Zuluftkanal 6 führt durch die beiden Gehäusehälften 34.

Ausgestaltungsvarianten des Schwenkhebels 60, ebenso wie die Montagemöglichkeiten des Dispensers lassen sich der WO 94/04280 entnehmen.

Der Erfindungsgegenstand ist zu demjenigen nach der WO 94/04280 soweit kompatibel, als sämtliche Montagebohrungen und Schwenkhebel-Befestigungen unverändert dazu passen. - Dies erlaubt einen individuellen Austausch, je nach spezifischer Verwendung bzw. Einsatzort.

Während die schlagartig austretenden Tröpfchenwolken nach EP -A1- 0 127 573 und WO 94/04280 innert einiger Zehntelssekunden aus den Spendern austreten, dreht der durch den Federmotor 43 angetriebene Ventilator 3 während 25 bis 28 Sekunden und verbreitet eine nur sanfte Strömung, welche sich auf natürliche Weise im Raum ausbreitet.

## Patentansprüche

1. Vorrichtung zur Abgabe von desodorierenden und/oder Gerüche neutralisierenden und/oder parfümierenden flüchtigen Stoffen, mit einem Auslösemechanismus, welcher über einen an einer Drehachse fixierten Schwenkhebel bedarfsweise betätigbar ist und eine vorgegebene Menge Wirkstoff freigibt und in die Umgebung verteilt, wobei ein Verdunstungs-Dispenser vorgesehen ist, welcher in wenigstens einer Vorratsflasche Wirkstoffe enthält, die über wenigstens ein Verdunstungselement in einem zumindest partiell geschlossenen Raum verdunsten und eine mit dem flüchtigen Wirkstoff angereicherte Atmosphäre bilden, wobei dieser Verdichter die angereicherte Atmosphäre intervallweise in die Umgebungsluft verströmt, wobei das Verdunstungselement (23) flächig ausgebildet ist, so dass sich im Raum (20) der resultierende Dampfdruck im Bereich des Sättigungsdampfdruckes der Stoffkomponenten einstellt, dass diese derart angereicherte Atmosphäre (A*) mittels des durch den Schwenkhebel (60) aktivierten Verdichters (3) mechanisch beschleunigt in die Umgebungsluft (A) verströmt, wobei der Verdichter (3) gleichzeitig eine gleiche Menge Luft in den Verdunstungsraum einlässt und wobei sich die angereicherte Atmosphäre (A*) im Bereich des Sättigungsdampfdruckes einstellt, bevor der Verdichter (3) erneut aktiviert ist.

2. Vorrichtung nach Anspruch 1, **dadurch gekennzeichnet, dass** ein Federmotor (43) vorgesehen ist, der durch den Schwenkhebel (60) in einer I. Position gespannt, in einer II. Position, durch eine auf den Verdichter (3) wirkende Drehbewegung entspannt und in einer III. Position in Ruhelage ist.

3. Vorrichtung nach Anspruch 1 oder 2, **dadurch gekennzeichnet, dass** der Federmotor (43) über ein Getriebe (36) an das Laufrad (11) des Verdichters (3) angeschlossen ist.

4. Vorrichtung nach Anspruch 3, **dadurch gekennzeichnet, dass** zwischen dem Federmotor (43) und dem Getriebe (36) ein Umschaltjoch (37) geschaltet ist, welches unabhängig vom Drehsinn des Schwenkhebels (60) über ein Zwischengetriebe (45) die vorgegebene Drehrichtung des Verdichters (3) sichert.

5. Vorrichtung nach Anspruch 1, **dadurch gekennzeichnet, dass** der Verdichter (3) ein Zug-/Druck- Ventilator ist, welcher saugseitig eine etwa gleiche Menge (q*) gesättigte Atmosphäre (A*) aus dem Verdunstungs-Raum (20) abführt und in die Umgebungsluft (A) ausbläst, die er druckseitig in den Verdunstungs-Raum (20) fördert.

6. Vorrichtung nach Anspruch 1, **dadurch gekennzeichnet, dass** der Verdunstungs-Raum (20) zur Aufnahme von zwei Vorratsflaschen (21, 22) mit Verdunstungselementen (23, 24) Raum bietet, wobei eine der beiden Flaschen (21) im Bereich einer Absaugöffnung (7) des Saugteils des Ventilators (3) plaziert ist.

7. Vorrichtung nach Anspruch 6, **dadurch gekennzeichnet, dass** der Absaugöffnung (7) eine einstellbare Blende (9) vorgelagert ist.

8. Vorrichtung nach Anspruch 1, **dadurch gekennzeichnet, dass** am oberen Teil eines Gerätegehäuses (25) ein Lufteinlass (5) vorgesehen ist.

9. Vorrichtung nach Anspruch 8, **dadurch gekennzeichnet, dass** im Gerätegehäuse (25) eine weitere Austrittsöffnung (8') vorgesehen ist, welche zum Verdunstungs-Raum (20) führt und einen Schieber (10) aufweist.

10. Vorrichtung nach Anspruch 1 bis 5, **dadurch gekennzeichnet, dass** der Zug-/Druck- Ventilator (3) ein einziges Laufrad (11) mit einer radialen Trennwand (13) aufweist, welche in zwei getrennten Kammern (14, 15) drehbar angeordnet ist.

11. Verfahren zum Betrieb einer Vorrichtung nach Anspruch 2, an einer Türe, wobei die Drehachse des Schwenkhebels in der Vorrichtung gelagert ist, **dadurch gekennzeichnet, dass** der Schwenkhebel (60) beim Schliessen der Türe am Türrahmen angelegt wird und dabei die I. Position einnimmt, dass beim Öffnen der Türe der Schwenkhebel (60) in die II. Position übergeführt wird und, dass sich der Schwenkhebel (60) bei geöffneter oder nur angelehnter Türe in seiner III. Position befindet.

12. Verfahren zum Betrieb einer Vorrichtung nach Anspruch 2, an einem Türrahmen, wobei die Drehachse des Schwenkhebels in der Vorrichtung gelagert ist, **dadurch gekennzeichnet, dass** der Schwenkhebel (60) beim Schliessen der Türe an dieser angelegt wird und dabei die I. Position einnimmt, dass beim Öffnen der Türe der Schwenkhebel (60) in die II. Position übergeführt wird und dass sich der Schwenkhebel (60) bei geöffneter oder nur angelehnter Türe in seiner III. Position befindet.

## Claims

1. Device for dispensing deodorizing and/or odour neutralizing and/or perfuming volatile substances, with a trigger mechanism which can be actuated, as required, by a pivoted lever fixed on a rotation axis, and releases a predetermined quantity of active agent and distributes this into the surroundings, where an evaporation dispenser is provided which contains active agents in at least one storage bottle, which active agents evaporate via at least one evaporating element in an at least partially closed space and form an atmosphere enriched with the volatile active agent, where this compressor makes the enriched atmosphere flow at intervals into the ambient air, where the evaporating element (23) is designed in a flat form, so that the resulting vapour pressure in the space (20) is in the range of the saturation vapour pressure of the components of the agent, where this enriched atmosphere (A*) is made to flow by means of the compressor (3) activated by the pivoted lever (60) into the ambient air (A) in a mechanically accelerated manner, where the compressor (3) simultaneously admits an equal quantity of air into the evaporation space, and where the enriched atmosphere (A*) is in the range of the saturation vapour pressure before the compressor (3) is activated again.

2. Device according to Claim 1, **characterized in that** a spring motor (43) is provided which is tensioned by the pivoted lever (60) into a Position I, relaxed in a Position II by a rotational movement acting on the compressor (3) and in a Position III, is in a resting position.

3. Device according to Claim 1 or 2, **characterized in that** the spring motor (43) is connected via a transmission (36) to the rotor (11) of the compressor (3).

4. Device according to Claim 3, **characterized in that** a commutation yoke (37) is connected between the spring motor (43) and the transmission (36), which commutation yoke secures the pre-set direction of rotation of the compressor (3) via an intermediate transmission (45), regardless of the direction of rotation of the pivoted lever (60).

5. Device according to Claim 1, **characterized in that** the compressor (3) is a suction/delivery fan, the suction side of which removes an approximately equal quantity (q*) of saturated atmosphere (A*) from the evaporation space (20) and discharges this into the ambient air (A), which ambient air the delivery side of the fan conveys into the evaporation space (20).

6. Device according to Claim 1, **characterized in that** the evaporation space (20) provides space for receiving two storage bottles (21, 22) with evaporating elements (23, 24), one of the two bottles (21) being placed in the region of a suction opening (7) of the suction part of the fan (3).

7. Device according to Claim 6, **characterized in that** an adjustable shutter (9) is positioned in front of the suction opening (7).

8. Device according to Claim 1, **characterized in that** an air inlet (5) is provided on the upper part of an instrument housing (25).

9. Device according to Claim 8, **characterized in that** in the instrument housing (25), a further outlet opening (8') is provided, which leads to the evaporation space (20) and which has a slider (10).

10. Device according to Claims 1 to 5, **characterized in that** the suction/delivery fan (3) has only one rotor (11) with a radial partition (13), which rotor is rotatably arranged in two separate chambers (14, 15).

11. Method for operating a device according to Claim 2 on a door, where the rotation axis of the pivoted lever is supported in the device, **characterized in that** the pivoted lever (60), on closing the door, is laid against the door frame and assumes the Position I, **in that** on opening the door, the pivoted lever (60) is moved into the Position II, and **in that** when the door is open or only ajar, the pivoted lever (6) is in its Position III.

12. Method for operating a device according to Claim 2 on a door frame, where the rotation axis of the pivoted lever is supported in the device, **characterized in that** on closing the door, the pivoted lever (60) is laid against the door and assumes the Position I, **in that** on opening the door, the pivoted lever (60) is moved into the Position II, and **in that** when the door is open or only ajar, the pivoted lever (60) is in its Position III.

## Revendications

1. Dispositif pour diffuser des produits volatiles désodorisants et/ou neutralisant des odeurs et/ou parfumants, présentant un mécanisme de déclenchement, lequel peut être actionné selon les besoins par l'intermédiaire d'un levier pivotant fixé à un axe de rotation et libère et répartit dans l'atmosphère une quantité donnée d'agent actif, dans lequel est prévu un diffuseur d'évaporation, lequel contient des principes actifs dans au moins un flacon de réserve qui s'évaporent dans un espace au moins partiellement fermé par l'intermédiaire d'au moins un élément d'évaporation et forment une atmosphère enrichie du principe actif volatile, dans lequel ce compresseur diffuse par intervalles l'atmosphère enrichie dans l'air ambiant, l'élément d'évaporation (23) étant réalisé surfacique de telle sorte qu'il se forme dans l'espace (20) la pression de vapeur résultante, dans le domaine de la pression de vapeur de saturation des composants du produits, de telle sorte que cette atmosphère ainsi enrichie (A*) se diffuse dans l'air ambiant (A) de manière accélérée mécaniquement au moyen du compresseur (3) activé par le levier pivotant (60), dans lequel le compresseur (3) laisse simultanément entrer une quantité égale d'air dans l'espace d'évaporation, dans lequel l'atmosphère enrichie (A*) se forme dans le domaine de pression de vapeur de saturation, avant que le compresseur (3) ne soit une nouvelle fois activé.

2. Dispositif selon la revendication 1, **caractérisé en ce qu'**un mouvement à ressort (43) est prévu, qui est armé dans une position I. par le levier pivotant (60), est détendu dans une position II. par l'intermédiaire d'un mouvement de rotation agissant sur le compresseur (3) et est en position de repos dans une position III.

3. Dispositif selon la revendication 1 ou 2, **caractérisé en ce que** le mouvement à ressort (43) est raccordé par l'intermédiaire d'un mécanisme (36) à la roue à ailettes (11) du compresseur (3).

4. Dispositif selon la revendication 3, **caractérisé en ce qu'**entre le mouvement à ressort (43) et le mécanisme (36) est montée une fourchette de commutation (37) laquelle, par l'intermédiaire d'un mécanisme intermédiaire (45), assure le sens de rotation donné du compresseur (3) indépendamment du sens de rotation du levier pivotant (60).

5. Dispositif selon la revendication 1, **caractérisé en ce que** le compresseur (3) est un ventilateur à aspiration/refoulement lequel, côté aspiration, évacue hors de l'espace d'évaporation (20) et souffle dans l'air ambiant (A) sensiblement une même quantité (q*) d'atmosphère saturée (A*), qu'il achemine côté pression dans l'espace d'évaporation (20).

6. Dispositif selon la revendication 1, **caractérisé en ce que** l'espace d'évaporation (20) offre de l'espace pour recevoir deux flacons de réserve (21, 22) présentant des éléments d'évaporation (23, 24), l'un des deux flacons (21) étant placé dans la zone d'une ouverture d'aspiration (7) de la partie aspiration du ventilateur (3).

7. Dispositif selon la revendication 6, **caractérisé en ce que** devant l'ouverture d'aspiration (7) est monté un obturateur (9) réglable.

8. Dispositif selon la revendication 1, **caractérisé en ce qu'**à la partie supérieure d'un boîtier d'appareil (25) est prévue une entrée d'air (5).

9. Dispositif selon la revendication 8, **caractérisé en ce que** dans le boîtier d'appareil (25) est prévue une autre ouverture de sortie (8'), laquelle mène à l'espace d'évaporation (20) et présente un registre (10).

10. Dispositif selon l'une des revendications 1 à 5, **caractérisé en ce que** le ventilateur à aspiration/refoulement (3) présente une roue à ailettes (11) unique, comportant une paroi de séparation radiale (13), laquelle est montée en rotation dans deux chambres séparées (14, 15).

11. Procédé pour faire fonctionner un dispositif selon la revendication 2, sur une porte, l'axe de rotation du levier pivotant étant supporté dans le dispositif, **caractérisé en ce que** le levier pivotant (60), lors de la fermeture de la porte, vient en appui sur l'encadrement de porte et adopte alors la position I., **en ce que** lors de l'ouverture de la porte, le levier pivotant (60) est amené dans la position II., et **en ce que** le levier pivotant (60) lorsque la porte est ouverte ou seulement rabattue se situe dans sa position III.

12. Procédé pour faire fonctionner un dispositif selon la revendication 2, sur une porte, l'axe de rotation du levier pivotant étant supporté dans le dispositif, **caractérisé en ce que** le levier pivotant (60), lors de la fermeture de la porte, vient en appui sur celle-ci et adopte alors la position I., **en ce que** lors de l'ouverture de la porte, le levier pivotant (60) est amené dans la position II., et **en ce que** le levier pivotant (60) lorsque la porte est ouverte ou seulement rabattue se situe dans sa position III.
